(19) Europäisches Patentamt
European Patent Office
Office européen des brevets

(11) Veröffentlichungsnummer : **0 177 681**
**B1**

# (12) EUROPÄISCHE PATENTSCHRIFT

(45) Veröffentlichungstag der Patentschrift : **28.09.88**

(51) Int. Cl.⁴ : **C 07 D233/90**

(21) Anmeldenummer : **85108552.2**

(22) Anmeldetag : **10.07.85**

## (54) Verfahren zur Herstellung von in 2-Stellung substituierten Imidazol-4, 5-dicarbonsäuren.

(30) Priorität : **17.07.84 DE 3426195**

(43) Veröffentlichungstag der Anmeldung : **16.04.86 Patentblatt 86/16**

(45) Bekanntmachung des Hinweises auf die Patenterteilung : **28.09.88 Patentblatt 88/39**

(84) Benannte Vertragsstaaten : **BE CH DE FR GB IT LI NL**

(56) Entgegenhaltungen :
**EP-A- 0 088 961**
**EP-A- 0 116 828**

(73) Patentinhaber : **BASF Aktiengesellschaft**
**Carl-Bosch-Strasse 38**
**D-6700 Ludwigshafen (DE)**

(72) Erfinder : **Kempe, Uwe, Dr.**
**Danziger Strasse 9**
**D-6701 Dannstadt-Schauernheim (DE)**
Erfinder : **Disteldorf, Walter, Dr.**
**Portugieser Weg 17**
**D-6706 Wachenheim (DE)**
Erfinder : **Dockner, Toni, Dr.**
**Grossgasse 6**
**D-6701 Meckenheim (DE)**
Erfinder : **Eisfeld, Wolfgang, Dr.**
**Dubliner Strasse 6**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 177 681 B1

**Beschreibung**

Diese Erfindung betrifft ein Verfahren zur Herstellung von in 2-Stellung substituierten Imidazol-4,5-dicarbonsäuren durch Oxidation der entsprechenden 4,5-Dihydroxymethylimidazole mit Salpetersäure.

In 2-Stellung substituierte 4,5-Imidazoldicarbonsäuren lassen sich z. B. durch Umsetzung von Weinsäurenitrat mit Aldehyden und Ammoniak herstellen. Nach dieser in K. Hofmann : Imidazole and its Derivatives, Part 1, 1953, Seiten 178-179 beschriebenen Synthese erhält man zwar gute Ausbeuten, der Einsatz von Weinsäurenitrat kompliziert aber die Durchführung und erfordert bei Ausübung des Verfahrens in einer technischen Anlage einen zu hohen Aufwand.

Man hat auch schon versucht, 2-substituierte Imidazolcarbonsäuren durch Oxidation zu erhalten, z. B. durch Behandlung von 2-substituierten Benzimidazolen mit Bichromat/Schwefelsäure (Z. obsz. Chem. 31. (1961), 1476-1479), mit Hydroperoxid (Arkiv Kemi 14, (1958) 419-428) oder mit Ozon in flüssiger Phase (Ukr. Khim Zh. 42 (1976) 394-398). Bei diesen Verfahren hat man es mit aufwendigen Ausgangsstoffen und Umweltproblemen zu tun ; auch ist ihre technische Durchführung schwierig und die Ausbeuten sind mäßig. Schließlich lassen sich Imidazolcarbonsäuren auch nach dem in der DE-PS 1 033 667 beschriebenen Verfahren aus den Imidazolen durch Carboxylierung gewinnen. Dieses Verfahren hat den Nachteil, daß hohe Drücke benötigt werden und die Ausbeuten nicht befriedigen.

Es wurde nun gefunden, daß man in 2-Stellung substituierte Imidazol-4,5-dicarbonsäuren der Formel

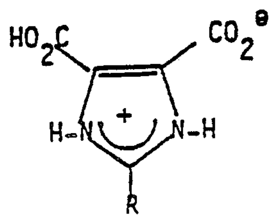

(I)

in der R einen Alkylrest mit 1 bis 17 C-Atomen, der noch Reste der Formel R'O—, $R'_2N$—, R'S— oder Arylgruppen enthalten kann, oder eine Arylgruppe bedeutet und R' für eine Alkylgruppe mit 1 bis 4 C-Atomen steht, vorteilhaft dadurch herstellen kann, daß man in 2-Stellung substituierte 4,5-Dihydroxymethylimidazole der Formel

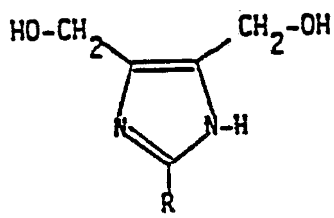

(II)

in der R die obengenannte Bedeutung hat, bei Temperaturen von 10 bis 130 °C mit Salpetersäure oxidiert, wobei man die Salpetersäure in mindestens stöchiometrischen Mengen anwendet.

Nach dem neuen Verfahren erhält man die in 2-Stellung substituierten Imidazol-4,5-dicarbonsäuren auf einfache Weise und in guten Ausbeuten. Dieses vorteilhafte Ergebnis ist überraschend, weil nach den Angaben in K. Hofmann : Imidazolen and its Derivatives, Part 1, (1953), Seiten 55 und 56 und in der DE-OS 32 12 749 zu erwarten war, daß die Hydroximethylimidazole durch die Behandlung mit Salpetersäure in die entsprechenden Aldehyde übergeführt werden. Außerdem konnte nicht ausgeschlossen werden, daß der Heterocyclus gespalten wird.

Die Bishydroximethylimidazole der Formel II enthalten in 2-Stellung z. B. einen Alkylrest mit 1 bis 17 C-Atomen, wie einen Methyl-, Ethyl-, Propyl-, Butyl- oder Heptadecylrest, oder einen Arylrest, wie einen Phenylrest. Der Alkylrest kann noch durch Alkylether-, Alkylthioether- oder Dialkylaminogruppen substituiert sein, in denen die Alkylreste z. B. 1 bis 4 C-Atome enthalten. Der Alkylrest kann auch durch Arylgruppen, wie Phenylreste substituiert sein. Beispielsweise seien die folgenden Bishydroximethylimidazole genannt : 2-Methyl-4,5-dihydroximethylimidazol, 2-Ethyl-4,5-dihydroximethylimidazol, 2-Isopropyl-4,5-dihydroximethylimidazol, 2-Methoximethyl-4,5-dihydroximethylimidazol und 2-Phenyl-4,5-dihydroximethylimidazol.

Man kann diese Ausgangsstoffe z. B. nach den Angaben der DE-OS 3 426 081 durch einfache Umsetzung von in 2-Stellung substituierten Imidazolen mit Formaldehyd in Gegenwart von basisch wirkenden Mitteln herstellen.

Nach dem erfindungsgemäßen Verfahren, das formelmäßig wie folgt beschrieben werden kann :

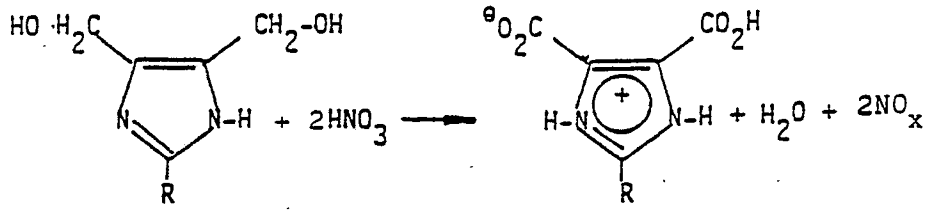

setzt man die in 2-Stellung substituierten 4,5-Dihydroximethylimidazole zweckmäßigerweise mit wäßriger Salpetersäure bei Temperaturen von 10 bis 130 °C um. Man verwendet z. B. 30 bis 80-%ige wäßrige Salpetersäure. Die Salpetersäure wird z. B. in mindestens stöchiometrischen Mengen angewendet. Vorzugsweise verwendet man die 5- bis 20-fache molare Menge Salpetersäure, bezogen auf das Dihydroximethylimidazol.

Als Lösungsmittel für die Oxidation kommen außer dem schon erwähnten Wasser z. B. auch Mischungen von Wasser mit Schwefelsäure, Benzolsulfonsäure, Toluolsulfonsäure oder einer aliphatischen Carbonsäure mit mindestens zwei C-Atomen, wie Essigsäure, in Betracht. Man kann die Umsetzung z. B. durch Zugabe von Katalysatoren beschleunigen. Als Katalysatoren sind z. B. Vanadationen geeignet, die z. B. in einer Menge von 0,003 bis 0,4, vorzugsweise 0,05 bis 0,09 Gew.% (berechnet auf V), bezogen auf die wäßrige Salpetersäure, eingesetzt werden.

Man führt die Oxidation z. B. so aus, daß man entweder die Salpetersäure vorlegt und die Imidazolverbindung zugibt, oder daß man die Imidazolverbindung, zweckmäßigerweise in einem der obengenannten wäßrigen Lösungsmittel vorlegt und die Salpetersäure zugibt. Zum Zwecke eines verzögerungsfreien Startens der Oxidation empfiehlt es sich, das zum Einleiten der Oxidation benötigte freie $NO_2$ durch Zugabe von Formaldehyd, Paraformaldehyd oder Trioxan zu erzeugen. Bei dieser Arbeitsweise gibt man, bezogen auf die wäßrige Salpetersäure, 0,03 bis 1, vorzugsweise 0,09 bis 0,11 Gew.% an Formaldehyd, Paraformaldehyd oder Trioxan zur vorgelegten Salpetersäure oder zur vorgelegten Imidazollösung.

Um einem zu starken Abfall der Salpetersäurekonzentration entgegenzuwirken, kann es von Vorteil sein, wenn man während der Reaktion Wasser abdestilliert. Das Verfahren der Erfindung kann kontinuierlich und diskontinuierlich durchgeführt werden. Bevorzugt wird bei Normaldruck gearbeitet, man kann aber auch unter vermindertem oder leichtem Überdruck oxidieren. Man arbeitet die Reaktionsgemische nach dem Ende der Oxidation z. B. dadurch auf, daß man sie aufkonzentriert und dann abkühlt. Dabei fallen die Endprodukte in sehr guten Ausbeuten und hoher Reinheit aus.

## Beispiel 1

7,1 g 2-Methyl-4,5-dihydroximethylimidazol werden in 70 ml 70 %iger Schwefelsäure unter Rühren auf 80 °C erhitzt. Nach Zugabe von 0,5 ml wäßrigem 30 %igem Formaldehyd werden in 15 Min. 25 g 63 %ige Salpetersäure gleichmäßig in das Gemisch gegeben. Zur Vervollständigung der Oxidation wird 1,75 h unter Reaktionsbedingungen gerührt. Das Reaktionsgemisch wird in 300 ml Wasser gegossen, der ausgefallene Feststoff gesammelt, mit Wasser neutralgewaschen und bei 60 °C im Vakuum getrocknet. Man erhält 7,1 g 2-Methylimidazol-4,5-dicarbonsäure als Monohydrat.

| | | | | |
|---|---|---|---|---|
| $C_6H_6O_4N_2 \cdot H_2O$ | Ber.: | C = 38,2 | H = 4,25 | 0 = 42,5 |
| | Gef.: | C = 38,5 | H = 4,3 | 0 = 42,5 |

## Beispiel 2

Beispiel 1 wird wiederholt, wobei man die Oxidation jedoch bei 30 °C durchführt und 8 h bei dieser Temperatur nachoxidiert. Man erhält 8,5 g 2-Methylimidazol-4,5-dicarbonsäuremonohydrat.

## Beispiel 3

Beispiel 1 wird wiederholt, wobei man die Oxidation jedoch bei 50 °C durchführt, anstelle der 70 %igen Schwefelsäure 50 ml Eisessig einsetzt und 20 h bei 20 °C nachrührt. Man erhält 8,1 g 2-Methylimidazol-4,5-dicarbonsäuremonohydrat.

## Beispiel 4

30 g 63 %ige Salpetersäure und 0,5 ml wäßriger 30 %iger Formaldehyd werden auf 60 °C erhitzt. In 15 Min. werden 7,1 g 2-Methyl-4,5-dihydroximethylimidazol zudosiert, danach wird noch 16 h bei 60 °C gerührt. Das Reaktionsgemisch wird in 300 ml Wasser gegossen, der Feststoff gesammelt, mit Wasser neutralgewaschen und bei 60 °C im Vakuum getrocknet. Man erhält 8,3 g 2-Methylimidazol-4,5-dicarbonsäuremonohydrat.

Beispiel 5

Man verfährt wie in Beispiel 4 beschrieben, wobei man die Oxidation jedoch in Gegenwart von 0,05 g Ammoniumnitrat durchführt und bei 60 °C nur 8 h nachoxidiert. Man erhält 8,4 g 2-Methylimidazol-4,5-dicarbonsäuremonohydrat.

Beispiel 6

7,1 g 2-Methyl-4,5-dihydroximethylimidazol werden in 20 ml Wasser mit 0,5 ml wäßrigem 30 %igem Formaldehyd auf 100 °C erhitzt. Zu dem Gemisch gibt man in 15 Min. 30 g 63 %ige Salpetersäure und rührt noch 8 h bei 100 °C nach. Es wird gemäß Beispiel 1 aufgearbeitet. Man erhält 8,2 g 2-Methylimidazol-4,5-dicarbonsäuremonohydrat.

Beispiel 7

In ein Gemisch aus 160 ml 63 %iger Salpetersäure und 80 ml Wasser werden 35,5 g 2-Methyl-4,5-dihydroximethylimidazol eingetragen. Das Reaktionsgemisch wird bei 55 °C 24 h gerührt. Aus dem Ansatz kristallisiert 2-Methylimidazol-4,5-dicarbonsäuremonohydrat in blütenweißen Kristallen aus. Nach dem Abfiltrieren und Trocknen erhält man 36,2 g, aus dem Filtrat nach Einstellen eines pH-Wertes von 3 weitere 2,3 g der Verbindung. Die Gesamtausbeute beträgt 82 %. Die Verbindung schmilzt bei 282 °C unter Zersetzung (Lit. : 320 bis 322 °C).

Beispiel 8

Man verfährt wie in Beispiel 7 beschrieben, wobei man jedoch 2-Ethyl-4,5-dihydroximethylimidazol einsetzt. Nach Durchführung der Reaktion werden 34,1 g 2-Ethylimidazol-4,5-dicarbonsäurehydrat erhalten, das entspricht einer Ausbeute von 67,5 %. Die Verbindung schmilzt bei 269 °C unter Zersetzung (Lit. : 259 °C).

Beispiel 9

Man verfährt wie in Beispiel 7 beschrieben, wobei man jedoch 42,5 g 2-Isopropyl-4,5-dihydroximethylimidazol einsetzt und das Reaktionsgemisch 30 h bei 55 °C rührt. Nach dem Abkühlen auf Raumtemperatur wird mit konzentrierter Natronlauge ein pH von 3 bis 4 eingestellt. 2-Isopropylimidazol-4,5-dicarbonsäure kristallisiert aus. Nach dem Abfiltrieren, Waschen mit Aceton und Trocknen erhält man 48,6 g dieser Substanz (Ausbeute 98,2 %), die bei 254 bis 255 °C unter Zersetzung schmilzt.

Beispiel 10

In eine Lösung aus 48 ml Wasser und 96 ml 68 %iger Salpetersäure werden 30,6 g 2-Phenyl-4,5-dihydroximethylimidazol langsam eingetragen. Man rührt das Gemisch bei 40 °C. Nach 15 Min. geht das Ausgangsmaterial in Lösung und der Ansatz verfärbt sich grünlich. Nach 15 h fällt das Produkt kristallin aus. Zur Vervollständigung der Reaktion wird 3 h bei 50 °C weitergerührt, dann abgekühlt und die ausfallende 2-Phenylimidazol-4,5-dicarbonsäure abfiltriert. Es wird dreimal mit je 50 ml Wasser und einmal mit 50 ml Aceton nachgewaschen und bei 60 °C getrocknet. Man erhält 20,8 g rein weißes kristallines Produkt (Ausbeute 59,8 %). Aus dem Filtrat fallen weitere 2,9 g des Produktes aus, nachdem ein pH-Wert von 8 bis 9 mit konzentrierter Natronlauge eingestellt wird (Gesamtausbeute 68,1 %). Die Substanz schmiltz bei 270, 5 °C unter Zersetzung.

**Patentansprüche**

1. Verfahren zur Herstellung von in 2-Stellung substituierten Imidazol-4,5-dicarbonsäuren der Formel

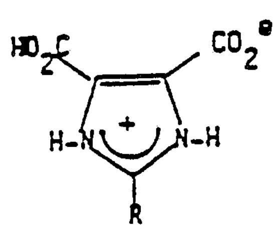

(I)

in der R einen Alkylrest mit 1 bis 17 C-Atomen, der noch Reste der Formel R'O—, $R'_2N$—, R'S— oder

Arylgruppen enthalten kann, oder eine Arylgruppe bedeutet und R' für eine Alkylgruppe mit 1 bis 4 C-Atomen steht, dadurch gekennzeichnet, daß man in 2-Stellung substituierte 4,5-Dihydroximethylimidazole der Formel

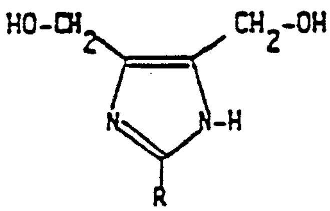

(II)

in der R die obengenannte Bedeutung hat, bei Temperaturen von 10 bis 130 °C mit Salpetersäure oxidiert, wobei man die Salpetersäure in mindestens stöchiometrischen Mengen anwendet.

2. Verfahren nach Anspruch 1, dadurch gekennzeichnet, daß man die 5- bis 20-fache molare Menge Salpetersäure, bezogen auf das 4,5-Dihydroximethylimidazol, anwendet.

3. Verfahren nach Anspruch 1, dadurch gekennzeichnet, daß man die Oxidation in Gegenwart von Wasser oder einer Mischung von Wasser mit Schwefelsäure, Benzolsulfonsäure, Toluolsulfonsäure oder einer aliphatischen Carbonsäure mit mindestens zwei C-Atomen durchführt.

4. Verfahren nach Anspruch 1, dadurch gekennzeichnet, daß man die Salpetersäure zur vorgelegten Imidazolverbindung oder die Imidazolverbindung zur vorgelegten Salpetersäure gibt.

5. Verfahren nach Anspruch 1, dadurch gekennzeichnet, daß man, bezogen auf die Salpetersäure, 0,03 bis 1 Gew.% an Formaldehyd, Paraformaldehyd oder Trioxan zum vorgelegten Reaktionspartner gibt.

**Claims**

1. A process for the preparation of 2-substituted imidazole-4,5-dicarboxylic acids of the formula

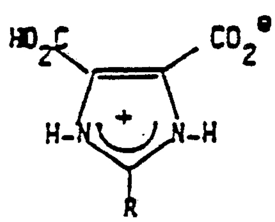

(I)

where R is alkyl of 1 to 17 carbon atoms which may furthermore contain radicals of the formula R'O—, $R'_2N$— or R'S— or aryl groups, or where R is aryl, and R' is alkyl of 1 to 4 carbon atoms, wherein a 2-substituted 4,5-dihydroxymethylimidazole of the formula

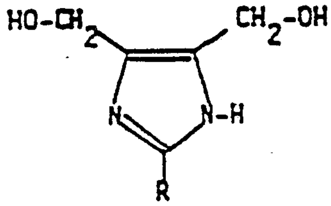

(II)

where R has the above meanings, is oxidized using nitric acid at from 10 to 130 °C, the nitric acid being used in a not less than stoichiometric amount.

2. A process as claimed in claim 1, wherein, based on the 4,5-dihydroxymethylimidazole, from 5 to 20 times the molar amount of nitric acid is used.

3. A process as claimed in claim 1, wherein the oxidation is carried out in the presence of water or a mixture of water with sulfuric acid, benzenesulfonic acid, toluenesulfonic acid or an aliphatic carboxylic acid of not less than two carbon atoms.

4. A process as claimed in claim 1, wherein the nitric acid is added to the initially charged imidazole compound or the imidazole compound to the initially charged nitric acid.

5. A process as claimed in claim 1, wherein from 0.03 to 1 % by weight, based on the nitric acid, of formaldehyde, paraformaldehyde or trioxane is added to whichever reactant constitutes the initial charge.

**Revendications**

1. Procédé de préparation d'acides imidazole-4,5-dicarboxyliques substitués en position 2 de formule

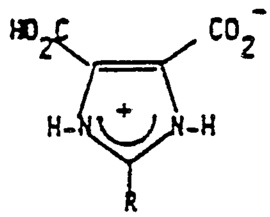

(I)

dans laquelle R est un reste alkyle ayant de 1 à 17 atomes de C, qui peut en outre comporter des restes de formule R'O—, $R'_2N$—, R'S— ou aryle, ou un reste aryle et R' est mis pour un groupement alkyle ayant de 1 à 4 atomes de C, caractérisé en ce qu'on oxyde un 4,5-dihydroxyméthylimidazole de formule

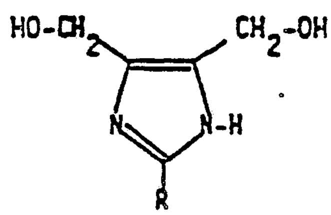

(II)

dans laquelle R est tel que défini ci-dessus, avec de l'acide nitrique à une température de 10 à 130 °C, en utilisant une quantité d'acide nitrique au moins stoechiométrique.

2. Procédé selon la revendication 1, caractérisé en ce qu'on utilise une quantité d'acide nitrique 5 à 20 fois molaire par rapport au 4,5-dihydroxyméthylimidazole.

3. Procédé selon la revendication 1, caractérisé en ce qu'on effectue l'oxydation en présence d'eau ou d'un mélange d'eau avec l'acide sulfurique, l'acide benzènesulfonique, l'acide toluènesulfonique ou un acide carboxylique aliphatique ayant au moins deux atomes de C.

4. Procédé selon la revendication 1, caractérisé en ce qu'on ajoute l'acide nitrique au dérivé d'imidazole préalablement préparé ou on ajoute le dérivé d'imidazole à l'acide nitrique préalablement préparé.

5. Procédé selon la revendication 1, caractérisé en ce qu'on ajoute aux constituants réactionnels préalablement préparés de 0,03 à 1 % en poids, par rapport à l'acide nitrique, de formaldéhyde, de paraformaldéhyde ou de trioxanne.